# EUROPÄISCHE PATENTSCHRIFT

(11) **EP 1 796 649 B1**
(45) Veröffentlichungstag und Bekanntmachung des Hinweises auf die Patenterteilung: **17.07.2013**
(21) Anmeldenummer: 05786848.1
(22) Anmeldetag: 17.08.2005
(51) Int. Cl.: A61K 9/50

(54) **NANOTRANSPORTSYSTEM MIT DENDRITISCHER ARCHITEKTUR**
NANOTRANSPORT SYSTEM HAVING A DENDRITIC ARCHITECTURE
SYSTEME DE NANOTRANSPORT A ARCHITECTURE DENDRITIQUE

(30) Priorität: 17.08.2004 DE 102004039875
(43) Veröffentlichungstag der Anmeldung: 20.06.2007
(73) Patentinhaber: Universität Dortmund, 44227 Dortmund (DE)
(72) Erfinder: HAAG, Rainer, 12209 Berlin (DE); RADOWSKI, Michal, 10713 Berlin (DE)
(74) Vertreter: Thiel, Christian
(86) Internationale Anmeldenummer: PCT/EP2005/008918
(87) Internationale Veröffentlichungsnummer: WO 2006/018295

(56) Entgegenhaltungen:
- EP-A- 1 034 839
- EP-A- 1 382 385
- WO-A-96/40264
- WO-A-2004/096140

## Beschreibung

Die Erfindung betrifft ein Nanotransportsystem mit dendritischer (baumartiger) Architektur sowie dessen Herstellungsverfahren und Verwendung.

Ein solches System kann zum Transport von Farbstoffen, Effektstoffen und vor allem von verschiedensten Wirkstoffen verwendet werden. Denn obwohl laufend neue pharmazeutische Wirkstoffe zur Behandlung der verschiedensten Krankheiten entwickelt und getestet werden, sind die Therapien nur bedingt erfolgreich. Ein Grund dafür liegt in der oftmals schlechten Bioverfügbarkeit und den zum Teil sehr starken Nebenwirkungen, die gerade bei der systemischen Behandlung von Krebserkrankungen nicht zu unterschätzen sind.

Eine Lösung bzw. Verbesserung dieses unbefriedigenden Zustandes ist das so genannte Pharmatargeting. Unter Pharmatargeting wird allgemein der selektive Transport von Therapeutika und/oder Diagnostika, im weiteren überwiegend als Pharmaka bezeichnet, zu einem zu therapierenden oder diagnostisch zu erfassenden Zielgewebe, -organ etc. im menschlichen oder tierischen Körper verstanden.

Mit Hilfe von Transportsystemen werden Pharmaka zielgerichtet an den Einsatzort gebracht, sodass eine Reduzierung der Dosierung sowie eine drastische Verringerung unerwünschter Nebenwirkungen ermöglicht werden. Hierbei soll das Transportsystem den eingeschlossenen Wirkstoff auf dem Weg vom Applikationsort bis zum Zielort von der Körperumgebung abschirmen und erst am Zielort selbst, also am kranken Organ oder den kranken Zellen, den Wirkstoff freisetzen. Somit werden die Pharmaka überwiegend dem erkrankten Zielgewebe zugeführt, und eine Beeinträchtigung des restlichen Organismus wird weitgehend vermieden.

Aus dem Stand der Technik sind verschiedene Transportsysteme bekannt, wie beispielsweise Liposomen, Polymermizellen, Polymerkonjugate oder einfache dendritische Kern-Schale-Architekturen.

Liposomale Transportsysteme bestehen meistens aus einer einzigen Bilayer-Schicht aus Phospholipidmembranen, welche in der Lage sind, Wirkstoffe in ihrem Inneren effizient aufzunehmen und somit eine höhere Wirkstoffkonzentration im Tumorgewebe anzureichern. Sie sind deshalb interessant, da hydrophile Arzneistoffe in das wässrige Innenvolumen und hydrophobe Arzneistoffe in die Lipidschichten eingebaut werden können. Vorteilhaft ist weiterhin die Schutzfunktion von verkapselten Wirkstoffen hinsichtlich des enzymatischen Abbaus während des Transports im Körper.

Somit ist insbesondere die Tumortherapie wirksamer bei gleichzeitig verminderten systemischen Nebenwirkungen. Die Wirksamkeit der Liposomen liegt einerseits in der höheren Halbwertzeit während der Blutzirkulation und somit einer veränderten Pharmakokinetik, und andererseits darin bevorzugt Wirkstoffe in Tumoren und nicht in normalem Gewebe zu akkumulieren. Diese Eigenschaft wird in der Literatur als "Enhanced Permeability and Retention Effect" (EPR) beschrieben und basiert darauf, dass durch ein schnelles und unkontrolliertes Wachstum von Tumoren deren Mikrovaskularisation unvollständig, d. h. löchrig, und das lymphatische System nur sehr bedingt angelegt ist. Liposomen können die Blutzirkulation bevorzugt in Tumorarealen durch Endothelzelllücken verlassen und in deren Interstitium akkumulieren. Dort angelangt entweichen mit der Zeit Wirkstoffmoleküle aus den Liposomen oder können in Form intakter Liposomen in die Tumorzelle gelangen, um wirksam zu werden.

Diesen Vorteilen stehen allerdings viele Nachteile gegenüber, denn Liposomenphospholipide sind denselben Abbaumechanismen ausgesetzt wie körpereigene Zellmembranlipide. Liposomale Transportsysteme sind nur begrenzt haltbar. Insbesondere durch die bei der intravenösen Vergabe der Wirkstoffe auftretenden Scherkräfte werden sie leicht zerstört.

Wünschenswert ist auch die aktive Einschleusung der Wirkstoffe durch die Zellmembran, denn viele Wirkstoffe können nicht passiv durch die Zellmembran diffundieren.

Mit Hilfe der Kopplung von rezeptorspezifischen Antikörpern oder deren Fragmenten an der Oberfläche von Liposomen soll dies erreicht werden. Diese so genannten Immunoliposomen erkennen spezifische Antigene an der Tumorzelloberfläche und werden nach Bindung durch aktive Endozytose von den Tumorzellen aufgenommen. Verabreichte Chemotherapeutika werden somit direkt und im Idealfall ausschließlich in Tumorzellen transportiert. Die therapeutische Wirksamkeit wird damit optimiert und die systemische Toxizität in Folge unspezifischer Aufnahme in andere Gewebe weiter vermindert.

Leider scheint dieses Vorgehen nicht immer zu funktionieren. Wahrscheinlich erkennt das Immunsystem den Antikörper als Fremdkörper und fängt das komplexe Transportsystem mitsamt dem Arzneistoff ab, bevor das Ziel erreicht ist oder die Wirkung einsetzen kann.

Polymermizellen sind physikalische Aggregate ambiphiler Makromoleküle, die sich in Wasser spontan durch Selbstorganisation bilden können. Meistens ist der innere Block unpolar oder ionisch und der äußere Block, der den Kern durch sterische Stabilisierung schützt, polar. Sie werden häufig zur Solubilisierung unpolarer Wirkstoffe mit begrenzter Löslichkeit in Wasser bzw. zum Transport von Oligonukleotiden verwendet.

Aufgrund ihrer nur physikalischen Wechselwirkungskräfte können sie jedoch bei Scherkräften oder anderen äußeren Einflüssen wie hoher Temperatur oder hohem Druck zerfallen. Dies ist z. B. häufig während der Sterilisation der Fall. Auch sind Polymermizellen weniger für die aktive Freisetzung verkapselter Moleküle geeignet, die durch externe Signale wie pH-Änderung ausgelöst wird.

Nanotransportsysteme mit einfacher dendritischer Kern-Schale Architektur ermöglichen ebenfalls gezielte Wirkstofftransporte. Im Unterschied zu physikalischen Aggregaten ambiphiler Moleküle können durch die kovalente Modifizierung dendritischer Makromoleküle mit entsprechender Schale stabile mizellartige Strukturen erhalten werden, welche für die nichtkovalente Verkapselung von Arzneistoffen geeignet sind. Diese weisen jedoch keine gute Abschirmung der verkapselten Pharmaka auf und sind aufgrund der eingeschränkten Polaritätsvorgaben nicht universell einsetzbar.

In Polymerkonjugaten sind Pharmaka über pH- bzw. enzymatisch spaltbare Linker kovalent gebunden. Sie können durch die den Tumor versorgenden Blutgefäße (Endothelzellen) in das Tumorgewebe eindringen und dieses abtöten. Nachteil hierbei ist ebenfalls die Scherwirkung, hervorgerufen durch die Applikationsform. Des weiteren bedeutet die aufwändige mehrstufige Synthese einen erhöhten Kostenfaktor.

Nachteile der bekannten Transportsysteme sind also Löslichkeitsprobleme bzw. Instabilität im Blutstrom, Oxidationsempfindlichkeit, Hydrolyseempfindlichkeit und enzymatische Empfindlichkeit. Insbesondere durch die Scherwirkung bei der intravenösen Vergabe der Wirkstoffe können diese zerstört werden. Die bekannten Transportsysteme sind nicht immer sterilisierbar. Viele dieser Transportsysteme sind nicht lange lagerfähig.

Ein weiterer Nachteil ist die notwendige Genehmigung für jede neue Kombination von Wirkstoff und Transportsystem. Zulassungsverfahren für Arzneimittel dauern bekanntlich sehr lange und sind zudem sehr kostenintensiv.

Aufgabe der Erfindung ist es daher, ein Transportsystem bereitzustellen, mit dem die vorgenannten Nachteile vermieden werden können.

Zur Lösung schlägt die Erfindung ein Nanotransportsystem der eingangs genannten Art vor, welches aus mindestens einem dendritischen Kern und aus mindestens zwei Schalen besteht, gemäß Anspruch 1.

Hochverzweigte baumartige Polymere, sogenannte Dendrimere, sind seit langem bekannt. Die bisherigen Forschungen in Bezug auf Kern-Schale Architekturen beschränkten sich allerdings auf einschalige Systeme (z. B. R. Haag et al., Angew. Chem. 116, 2004, S. 280 - 284, bzw. 114, 2002, S. 4426 - 4431 sowie WO 02/077037 A2, WO 03/037383 A1 und US 2002/0187199 A1).

EP 1 382 385 A beschreibt ein Nanotransportsystem, bei dem ein dendritisches Kemmolekül mit einer Vielzahl von funktionellen Gruppen ausgestattet ist, die über Linker mit weiteren mehrfach funktionalisierten Verbindungen umgesetzt sind.

Nanotransportsystem, Nanotransporter, Multischalen-Nanotransporter, Nanocarrier und Transporter werden hier als Synonyme verwendet. Unter Wirkstoffen werden sowohl Pharmaka, Arzneistoffe, Antigene, Antikörper, Peptide, Nukleinsäuren, Nukleotide, Farbstoffe, Effektstoffe als auch Toxine verstanden.

Im Gegensatz zum Stand der Technik ist die Abschirmung der eingeschlossenen Wirkstoffe bei dem erfindungsgemäßen Nanotransportsystem besonders effektiv und effizient. Der Wirkstoff kann somit das Zielgewebe, wie z. B. kranke Zellen und Organe, unverändert erreichen. Aufgrund der kovalent verknüpften Multischalenstruktur zerfällt das Gerüst nicht bei äußeren Einflüssen wie z. B. Scherung bzw. Sterilisation, wie dies bei physikalischen Aggregaten der Fall sein kann.

Vorzugsweise weisen die Schalen unterschiedliche Polaritäten auf, somit wird ein Polaritätsgradient erreicht, um sowohl unpolare als auch polare Wirkstoffe einschließen zu können. Das erfindungsgemäße Nanotransportsystem weist daher eine mehrschalige unimolekulare Struktur auf. Durch die Kombination der verschiedenen Schalen ist es erstmalig möglich, immer wieder neue auf den Wirkstoff und die Verwendung abgestimmte Nanotransportsysteme zu kreieren. Die Variationen sind beinahe grenzenlos.

Bevorzugt weist das erfindungsgemäße Nanotransportsystem polare und unpolare Schalen auf, die in abwechselnder Reihenfolge angeordnet sind. Überraschenderweise wurde hierbei gefunden, dass die erfindungsgemäßen Multischalen-Nanotransporter sowohl polare als auch unpolare Wirkstoffe transportieren können.

Es ist jedoch auch vorstellbar, die Schalen hinsichtlich der Wirkstoffe und des Einsatzes zweckdienlich in andere Reihenfolge anzuordnen.

Vorzugsweise weist das System eine unpolare innere Schale und eine polare äußere Schale auf. Das erfindungsgemäße Nanotransportsystem ist kein rigides Gerüst. Durch Einstülpung bzw. Umlagerung der Schichtstruktur können unpolare Wirkstoffe in organischen Lösungsmitteln transportiert werden. Besonders bevorzugt ist der Transport von polaren Wirkstoffen in polaren Lösungen, wie z. B. Blut. Polare Wirkstoffe können derart geschützt in der Blutzirkulation verweilen, bis sie das Zielgewebe erreichen und ihre therapeutische Wirkung entfalten.

Für die Verkapselung der Wirkstoffe ist zentral ein dendritischer Kern angelegt, der aus dendritischen Polymeren besteht.

Unter dendritischen Polymeren werden sowohl Dendrimere als auch hyperverzweigte Polymere verstanden. Dendrimere haben aufgrund des stufenweisen Aufbaus eine perfekte baumartige Struktur. Deren Synthese ist aufwändig und daher mit hohen Kosten verbunden.

Für das erfindungsgemäße Nanotransportsystem können sowohl Dendrimere als auch hyperverzweigte Polymere eingesetzt. Vorteilhaft ist somit auch der Einsatz von hyperverzweigten Polymeren, sodass hier der Syntheseaufwand und somit auch die Kosten herabgesetzt werden.

Es hat sich überraschenderweise herausgestellt, dass eine Funktionalisierung des dendritischen Polymers von weniger als 100 %, vorzugsweise von 50 %, ausreicht. Dies bedeutet, dass der innere Kern des Polymers nicht voll funktionalisiert werden muss, um Wirkstoffe verkapseln zu können.

Vorzugsweise besteht der dendritische Kern aus Polyglycerin, Polyamid, Polyamin, Polyether oder Polyester. Diese Verbindungen können innerhalb der dendritischen Architektur auch weiter modifiziert werden. Der dendritische Kern kann somit gemäß seiner Modifikation polarisiert sein.

Vorzugsweise besteht die innere Schale aus langkettigen oder cyclischen Alkylketten mit einer Kohlenstofflänge von C₂ - C₄₀, besonders bevorzugt mit einer Kohlenstofflänge von C₁₂ - C₄₀, aromatischen Ringen oder Steroiden. Unpolare Verbindungen werden hier eingesetzt, um einen Polaritätsgradient zu erzeugen. Sie schirmen den Wirkstoff wirkungsvoll ab.

Bevorzugt besteht die äußere Schale aus Poly(ethylenglycol) mit der Strukturformel (-CH₂CH₂O-)ₙ, wobei n = 4 -150 ist, linearen oder verzweigten Polyglycerinen (C₃H₅OH)ₙ mit n = 2 - 150 , Polyglycerin-Dendronen [G1 - G6], Polyethern, Sacchariden oder Polyestern. Die polare Schale ermöglicht die Löslichkeit in polaren Lösungsmitteln.

Vorzugsweise ist die äußere Schale biokompatibel. Die "Tarneigenschaft" ist besonders wichtig, da dadurch das erfindungsgemäße Nanotransportsystem im Blutstrom vom Immunsystem nicht als Fremdkörper erkannt wird. Des Weiteren weist sie keine Wechselwirkung mit Proteinen auf bzw. sollte proteinabweisend sein, damit der Transport zu den Tumorzellen ermöglicht werden kann.

Mittels eines Linkers 1 ist die innere Schale mit dem dendritischen Kern verbunden. Dieser Linker sind enzymatisch spaltbar, säurelabil, photolabil oder thermolabil. Diese Eigenschaften ermöglichen somit die spezifische Spaltung der Schalen und damit auch die kontrollierte Wirkstoff-Freisetzung am Zielort aufgrund biochemischer und physiologischer Besonderheiten des pathogenen Gewebes. Besonders effizient für den Wirkstofftransport in biologischen Systemen ist die Freisetzung von eingeschlossenen Molekülen auf der Basis schwacher externer Signale, wie z. B. einer pH-Emiedrigung in Tumor- oder infizierten Geweben bei pH 5 bis 6.

Vorzugsweise besteht der Linker 1 aus Ester-, Amid-, Acetal-, Imin-, Disulfid- oder Hydrazongruppen. Diese Gruppen können durch externe Signale den Wirkstoff freisetzen.

Des weiteren ist die äußere Schale über einem Linker 2 mit der inneren Schale verbunden. Diese ist ebenfalls enzymatisch spaltbar, säurelabil, photolabil oder thermolabil, wobei der Linker 2 vorzugsweise etwas stabiler als der Linker 1 ist. Besonders bevorzugt besteht der Linker 2 aus Ester-, Amid-, Acetal-, Disulfid- oder Ethergruppen, da diese die geforderten Eigenschaften aufweisen.

Die äußere Schale besteht bevorzugt aus wasserlöslichen Einheiten und kann funktionelle Gruppen enthalten. Die funktionellen Gruppen sind vorzugsweise Hydroxy- oder Alkoxygruppen, besonders bevorzugt Amine, Amide oder Thiole. Gezielter Wirkstofftransport mittels aktiven Targetings durch Kopplung mit dirigierenden Gruppen, insbesondere mit Oligosacchariden, Peptiden, Antikörpern und (Oligo-)Nukleotiden, ist ebenfalls möglich.

Es sind jedoch auch andere enzymatisch spaltbare, säurelabile, photolabile oder thermolabile Verbindungen und funktionale Gruppen vorstellbar.

Des weiteren kann das System mehrere dendritische Kerne aufweisen, sodass verschiedene Wirkstoffe komplexiert bzw. eingeschlossen werden können.

Das erfindungsgemäße Nanotransportsystem ist unimolekular oder liegt in definierten Aggregaten vor. Der Vorteil ist daher eine definierte Struktur.

Das erfindungsgemäße Nanotransportsystem ist 3 - 50 nm, vorzugsweise 3 - 25 nm groß. Eine Bildung von größeren Aggregaten ist aber auch möglich. Bei Verkapselung bestimmter Stoffe konnte auch größere, reproduzierbare Aggregaten von 100 - 200 nm beobachtet werden.

Die Größe der Nanotransportsysteme ist von besonderer Bedeutung, damit diese einerseits von der Niere ausgeschieden (beispielsweise nach Degradation) und andererseits durch passives Targeting im Zielgewebe akkumuliert werden können. Nanopartikel, die größer sind als 5 nm, können biologische Barrieren im Vergleich zu kleineren Molekülen über andere Mechanismen passieren und damit die Spezifität von Wirkstoffen für bestimmtes Gewebe verbessern.

Es ist möglich, die Größe und Architektur des erfindungsgemäßen Nanotransporters verfahrenstechnisch zu definieren. Hinsichtlich des modularen Aufbaus sind zahlreiche Variationen herstellbar.

Der dendritische Kern beträgt vorzugsweise 3 - 15 nm. Diese Größe ist erforderlich, um Wirkstoffe zu komplexieren bzw. einzuschließen und somit zu transportieren.

Darüber hinaus betrifft die Erfindung ein Verfahren zur Herstellung von Nanotransportsystemen mit dendritischer Architektur mit mindestens zwei Schalen, wobei der modulare Aufbau der Schalen bevorzugt von außen nach innen erfolgt. Nach Bedarf kann der Aufbau der Schalen auch von innen nach außen stattfinden.

Der Einsatz des erfindungsgemäßen Nanotransporters ist in vielen Bereichen denkbar. Er ist nicht nur auf den Transport von Wirkstoffen beschränkt, sondern universell für alle Bereiche, bei denen Stoffe vor äußeren Einflüssen abgeschirmt und erst am Einsatzort wieder freigesetzt werden sollen, einsetzbar.

Des Weiteren betrifft die Erfindung die Verwendung des Nanotransportsystems zum Transport, der Solubilisierung bzw. Kompartimentierung von pharmazeutischen Wirkstoffen, insbesondere Chemotherapeutika und Zytostatika, Farb- bzw. Effektstoffen und Katalysatoren.

Somit ist die Verwendung des Nanotransportsystems zur zielgerichteten Freisetzung von pharmazeutischen Wirkstoffen, insbesondere in der Krebstherapie möglich.

Auch die Verwendung in der Gentherapie bzw. Gentechnologie, in der Diagnostik und in der Analytik ist vorstellbar.

Eine besondere Verwendung des Nanotransportsystems ist der Transport von si-RNA. Diese so genannten "small interfering RNA" sind kleine doppelsträngige RNA-Fragmente, welche gezielt funktionelle biologische Schritte (Proteinsynthese) in der Zelle unterbinden und damit zellbiologische Signalwege offen legen. Das geschieht dadurch, dass sich sehr kurze RNA-Sequenzen von 21 - 25 Nukleotiden (si-RNA = small interfering RNA) an die komplementäre mRNA heften und dadurch ihre Translation verhindern.

Hervorzuheben ist des Weiteren der Vorteil, dass nur eine einmalige Zulassung für den erfindungsgemäßen Nanotransporter erforderlich ist, um bereits zugelassene Wirkstoffe zu transportieren.

Insbesondere auch für den Transport von Stoffen, die für die Diagnostik oder Analytik von Bedeutung sind.

Nachstehend wird anhand von Beispielen die Erfindung näher erläutert.
- Fig. 1: schematische Darstellung eines erfindungsgemäßen multischalen Nanotransporters.
- Fig. 2: Tabelle bevorzugter Verbindungen
- Fig. 3: Reaktionsgleichung zur Herstellung von erfindungsgemäßen Nanotransportsystemen

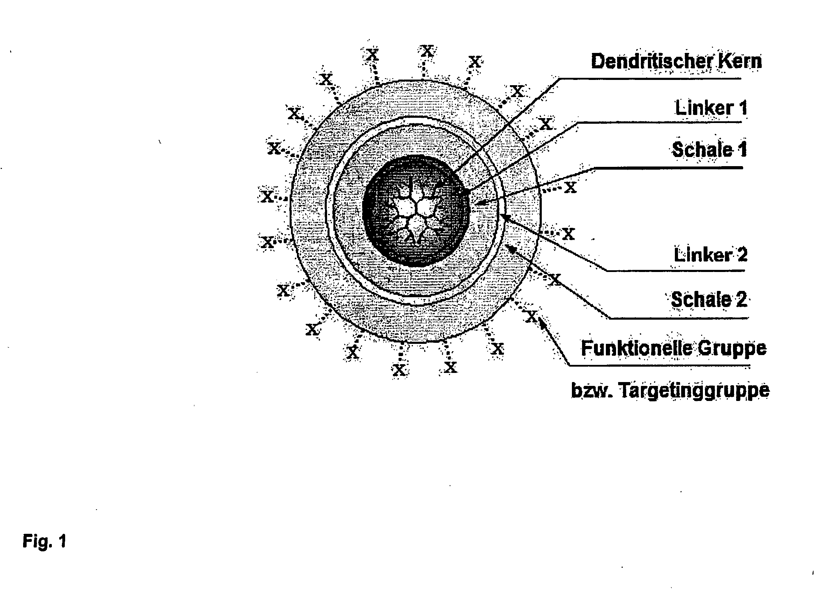

Fig. 1 beschreibt einen erfindungsgemäßen Multischalen-Nanotransporter mit einem inneren dendritischen Kern, wobei er mittels eines Linkers 1 mit einer inneren Schale 1 verbunden ist. Die innere Schale 1 ist mit der äußeren Schale 2 durch einen Linker 2 verbunden, wobei die äußere Schale 2 mit funktionellen Gruppen bzw. Targetinggruppen modifiziert ist. Der erfindungsgemäße Nanotransporter weist eine gute Abschirmung der eingeschlossenen Wirkstoffe, eine gute Komplexierung durch den Polaritätsgradient und eine hohe Flexibilität durch die modulare Bauweise auf.

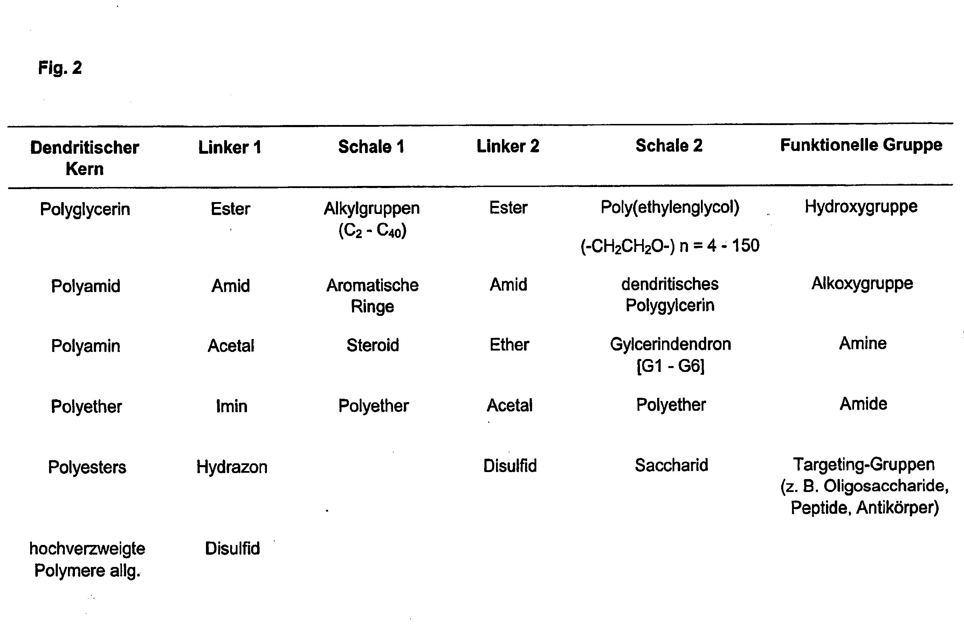

Fig. 2 stellt tabellarisch die in Frage kommenden Verbindungen für den erfindungsgemäßen Nanotransporter dar. Andere Verbindungen sind ebenfalls vorstellbar.

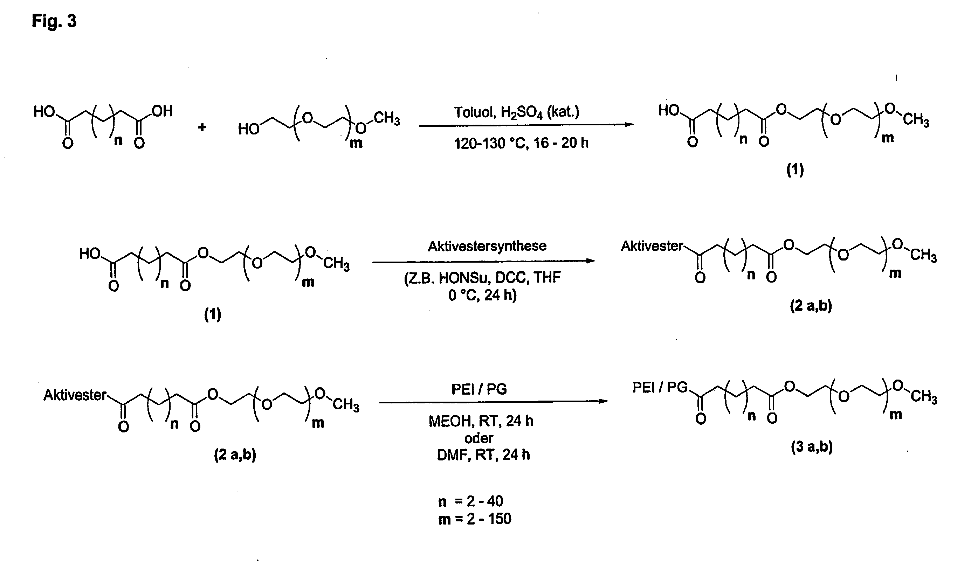

Fig. 3 zeigt anhand einer Reaktionsgleichung zwei Beispiele A und B für die Herstellung des erfindungsgemäßen Nanotransporters.

### Arbeitsvorschriften

### Beispiel A

Zunächst werden Poly(ethylenglycol)monomethylether (z. B. PEG 350, 550, 750) mit 1,18-Octadecandisäure in heißem Toluol (80 - 100 °C) versetzt. Das Reaktionsgemisch wird dann auf 120 - 130 °C erhitzt und für 20 Stunden azeotropisch destilliert. Als Katalysator wird Schwefelsäure verwandt. Nach dem Entfernen des Toluols und mehrstufigen Reinigungsschritten erhält man ein weißes/hellgelbes Wachsprodukt, nämlich die monoPEGylierte 1,18-Octadecandisäure (1). Dieses wird nun mit THF versetzt und auf 0 °C heruntergekühlt. Anschließend werden N,N'-Dicyclohexylcarbodiimid (DCC) und N-Hydroxysuccinimid hinzugefügt. Das Reaktionsgemisch wird bei etwa 0 °C für eine Stunde gerührt und dann bei 4 °C für 24 Stunden stehen gelassen. Nach mehreren Reinigungen erhält man ein weißes/hellgelbes Wachsprodukt, nämlich den MonoPEGyliertendisuccinimidester (2a). Nun werden hyperverzweigtes Polyethylenimin (PEI) und MonoPEGylierterdisuccinimidester (50% bezogen auf NH-Gruppen) mit Methanol versetzt und bei 0 °C für 1 Stunde und für weitere 24 Stunden bei Raumtemperatur gerührt. Man erhält nach Dialyse in Methanol und Verdampfen des Lösungsmittels ein farbloses Öl, nämlich den erfindungsgemäßen dendritischen Multischalen-Nanotransporter (3a).

### Beispiel B

Alternativ kann die monoPEGylierte 1,18-Octadecandisäure (1) in wasserfreiem THF mit N,N-Carbonyldiimidazol bei 30 °C für 1 h und 20 min bei 45 °C umgesetzt werden. Das Lösungsmittel wird im Vakuum entfernt und ein gelbliches Wachs (2b) erhalten, das ohne weitere Aufreinigung zur Kupplung mit dem hyperverzweigten Polyglycerin eingesetzt wurde. Dazu wurde eine Reaktionsmischung aus 2b (50% bezogen auf PG-OH-Gruppen) mit Polyglycerin in wasserfreiem DMF über Nacht unter Argon gerührt und anschließend für 6 h auf 60 °C erwärmt. Nach Entfernen des Lösungsmittels wurde das Rohprodukt (3b) durch Dialyse in Methanol gereinigt.

## Patentansprüche

1. Nanotransportsystem mit dendritischer (baumartiger) Architektur, wobei das System aus einem dendritischen Kern und aus mindestens zwei Schalen besteht, **dadurch gekennzeichnet, dass** das System unimolekular ist, wobei eine innere Schale mittels eines Linkers 1 mit dem dendritischen Kern verbunden ist und eine äußere Schale mittels eines Linkers 2 mit der inneren Schale verbunden ist.

2. Nanotransportsystem nach Anspruch 1, **dadurch gekennzeichnet, dass** die Schalen unterschiedliche Polaritäten aufweisen.

3. Nanotransportsystem nach einem der vorstehenden Ansprüche, **dadurch gekennzeichnet, dass** das System polare und unpolare Schalen aufweist.

4. Nanotransportsystem nach einem der vorstehenden Ansprüche 1 bis 3, **dadurch gekennzeichnet, dass** das System eine unpolare innere Schale und eine polare äußere Schale aufweist.

5. Nanotransportsystem nach einem der vorstehenden Ansprüche 1 bis 3, **dadurch gekennzeichnet, dass** das System eine polare innere Schale und eine unpolare äußere Schale aufweist.

6. Nanotransportsystem nach einem der vorstehenden Ansprüche 1 oder 2, **dadurch gekennzeichnet, dass** die innere und äußere Schale polar sind.

7. Nanotransportsystem nach einem der vorstehenden Ansprüche 1 oder 2, **dadurch gekennzeichnet, dass** die innere und äußere Schale unpolar sind.

8. Nanotransportsystem nach einem der vorstehenden Ansprüche, **dadurch gekennzeichnet, dass** das System einen dendritischen Kern aufweist, wobei dieser aus dendritischen Polymeren besteht.

9. Nanotransportsystem nach einem der vorstehenden Ansprüche, **dadurch gekennzeichnet, dass** der dendritische Kern aus Polyglycerin, Polyamid, Polyamin, Polyether oder Polyester besteht.

10. Nanotransportsystem nach einem der vorstehenden Ansprüche, **dadurch gekennzeichnet, dass** der dendritische Kern gemäß seiner Modifikation polarisiert ist.

11. Nanotransportsystem nach Anspruch 1, **dadurch gekennzeichnet, dass** die innere Schale aus langkettigen oder cyclischen Alkylketten, vorzugsweise mit einer Kohlenstofflänge von C₂ - C₄₀, besonders bevorzugt C₁₂ - C₄₀, aromatischen Ringen oder Steroiden besteht.

12. Nanotransportsystem nach Anspruch 1, **dadurch gekennzeichnet, dass** die außere Schale aus Poly(ethylenglycol) mit der Strukturformel (-CH₂CH₂O-)ₙ mit n = 3 - 150, linearem oder verzweigtem Polyglycerin (C₃H₅OH)ₙ mit n = 2 - 150, Polyglycerin-Dendrone [G1 - G6], Polyether, Sacchariden oder Polyester besteht.

13. Nanotransportsystem noch einem der vorstehenden Ansprüche, **dadurch gekennzeichnet, dass** die äußere Schale biokompatibel ist.

14. Nanotransportsystem nach einem der vorstehenden Ansprüche, **dadurch gekennzeichnet, dass** der Linker 1 enzymatisch spaltbar, säurelabil, photolabil oder thermolabil ist.

15. Nanotransportsystem nach einem der vorstehenden Ansprüche, **dadurch gekennzeichnet, dass** der Linker 1 aus Ester-, Amid-, Acetal-, Imin-, Disulfid- uder Hydrazongruppen besteht.

16. Nanotransportsystem nach einem der vorstehenden Ansprüche, **dadurch gekennzeichnet, dass** der Linker 2 enzymatisch spaltbar, säurelabil, photolabil oder thermolabil ist.

17. Nanotransportsystem nach einem der vorstehenden Ansprüche, **dadurch gekennzeichnet, dass** der Linker 2 aus Ester-, Amid-, Acetal-, Disulfid- oder Ethergruppen besteht.

18. Nanotransportsystum nach einem der vorstehenden Ansprüche, **dadurch gekennzeichnet, dass** die äußere Schale durch funktionelle Gruppen terminiert ist.

19. Nanotransportsystem nach Anspruch 18, **dadurch gekennzeichnet, dass** die terminalen funktionellen Gruppen vorzugsweise Hydroxy- oder Alkoxygruppen, besonders bevorzugt Amine, Amide oder Thiole sind.

20. Nanotransportsystem nach einem der vorstehenden Ansprüche, **dadurch gekennzeichnet, dass** die terminalen funktionellen Gruppen eine dirigierende Funktion aufweisen.

21. Nanotransportsystem nach Anspruche 20, **dadurch gekennzeichnet, dass** die terminalen dirigierenden Gruppen vorzugsweise Peptide, Antikörper, Oligosaccharide, Nukleinsäuren oder Nukleotide sind.

22. Nanotransportsystem nach einem der vorstehenden Ansprüche, **dadurch gekennzeichnet, dass** das System polare und/oder unpolare Stoffe aufnimmt.

23. Nanotransportsystem nach einem der vorstehenden Ansprüche, **dadurch gekennzeichnet, dass** das System aggregiert.

24. Nanotransportsystem nach einem der vorstehenden Ansprüche, **dadurch gekennzeichnet, dass** das System 3 - 50 nm, vorzugsweise 3 - 25 nm, groß ist.

25. Nanotransportsystem nach einem der vorstehenden Ansprüche, **dadurch gekennzeichnet, dass** der dendritische Kern 3 -15 nm groß ist.

26. Nanotransportsystem nach Anspruch 25, **dadurch gekennzeichnet, dass** das System größer als 15 nm ist.

27. Verfahren zur Herstellung von Nanotransportsystemen mit dendritischer Architektur nach einem der Ansprüche 1 bis 26, **dadurch gekennzeichnet, dass** der modulare Aufbau der Schalen von außen nach innen erfolgt.

28. Verwendung des Nanotransportsystems zum Transport von pharmazeutischen Wirkstoffen, insbesondere Chemotherapeutika, Zytostatika, Antikörpern, Peptiden und Nukleinsäuren.

29. Verwendung des Nanotransportsystems nach einem der Ansprüche 1 bis 26 zur Herstellung eines Mittels zum Transport von chemischen Stoffen, insbesondere von Farbstoffen oder Effektstoffen.

30. Verwendung des Nanotransportsystems nach einem der Ansprüche 1 bis 26 zur Herstellung eines Mittels zur zielgerichteten Freisetzung von pharmazeutischen Wirkstoffen, insbesondere in der Krebstherapie.

31. Verwendung des Nanotransportsystems nach einem der Ansprüche 1 bis 26 zur Herstellung eines Mittels, für die Gentherapie, die Diagnostik und die Analytik.

32. Verwendung des Nanotransportsystems nach einem der Ansprüche 1 bis 26 zur Herstellung eines Mittels zum Transportieren von si-RNA oder anderen Nukleinsäure-Derivaten.

33. Verwendung des Nanotransportsystems nach einem der Ansprüche 1 bis 26 zur Herstellung eines Mittels zum Transport von radioaktiv markierten Wirkstoffen oder Molekülen.

34. Verwendung des Nanotransportsystems nach einem der Ansprüche 1 bis 26 zur Herstellung eines Mittels zur Applikation im Menschen und im Tier, insbesondere zur intravenösen Applikation.

35. Verwendung des Nanotransportsystems in der Diagnostik außerhalb des menschlichen oder tierischen Körpers.

## Claims

1. A nanotransport system with dendritic (tree-like) architecture, wherein the system consists of a dendritic core and at least two shells, **characterised in that** the system is unimolecular, wherein an inner shell is connected to the dendritic core by means of a linker 1 and an outer shell is connected to the inner shell by means of a linker 2.

2. A nanotransport system as claimed in claim 1, **characterised in that** the shells have different polarities.

3. A nanotransport system as claimed in one of the preceding claims, **characterised in that** the system has polar and nonpolar shells.

4. A nanotransport system as claimed in one of the preceding claims 1 to 3, **characterised in that** the system has a nonpolar inner shell and a polar outer shell.

5. A nanotransport system as claimed in one of the preceding claims 1 to 3, **characterised in that** the system has a polar inner shell and unipolar outer shell.

6. A nanotransport system as claimed in one of the preceding claims 1 or 2, **characterised in that** the inner and outer shells are polar.

7. A nanotransport system as claimed in one of the preceding claims 1 or 2, **characterised in that** the inner and outer shells are nonpolar.

8. A nanotransport system as claimed in one of the preceding claims, **characterised in that** the system has a dendritic core, wherein it consists of dendritic polymers.

9. A nanotransport system as claimed in one of the preceding claims, **characterised in that** the dendritic core consists of polyglycerine, polyamide, polyamine, polyether or polyester.

10. A nanotransport system as claimed in one of the preceding claims, **characterised in that** the dendritic core is polarised in accordance with its modification.

11. A nanotransport system as claimed in claim 1, **characterised in that** the inner shell consists of long chain or cyclic alkyl chains, preferably with a carbon length of C₂-C₄₀, preferably C₁₂-C₄₀, aromatic rings or steroids.

12. A nanotransport system as claimed in claim 1, **characterised in that** the outer shell consists of poly(ethyleneglycol) with the structural formula (-CH₂CH₂O-)ₙ, where n = 3 - 150, linear or branched polyglycerine (C₃H₅OH)ₙ, where n =2 - 150, polyglycerine dendrons [G1-G6], polyethers, saccharides or polyester.

13. A nanotransport system as claimed in one of the preceding claims, **characterised in that** the outer shell is biocompatible.

14. A nanotransport system as claimed in one of the preceding claims, **characterised in that** the linker 1, is enzymatically dissociable, acid-labile, photolabile or thermo-labile.

15. A nanotransport system as claimed in one of the preceding claims, **characterised in that** the linker 1 consists of ester, amide, acetal, imine, disulphide or hydrazone groups.

16. A nanotransport system as claimed in one of the preceding claims, **characterised in that** the linker 2 is enzymatically dissociable, acid-labile, photolabil or thermolabil.

17. A nanotransport system as claimed in one of the preceding claims, **characterised in that** the linker 2 consists of ester, amide, acetal, disulphide or ether groups.

18. A nanotransport system as claimed in one of the preceding claims, **characterised in that** the outer shell is terminated by functional groups.

19. A nanotransport system as claimed in claim 18, **characterised in that** the terminal functional groups are preferably hydroxyl or alkoxy groups, particularly preferably amines, amides or thiols.

20. A nanotransport system as claimed in one of the preceding claims, **characterised in that** the terminal functional groups have a directional function.

21. A nanotransport system as claimed in claim 20, **characterised in that** the terminal directional groups are preferably peptides, antibodies, oligosaccharides, nucleic acids or nucleotides.

22. A nanotransport system as claimed in one of the preceding claims, **characterised in that** the system absorbs polar and/or nonpolar substances.

23. A nanotransport system as claimed in one of the preceding claims, **characterised in that** the system aggregates.

24. A nanotransport system as claimed in one of the preceding claims, **characterised in that** the system is 3 - 50 nm, preferably 3 - 25 nm, large.

25. A nanotransport system as claimed in one of the preceding claims, **characterised in that** the dendritic core is 3 -15 nm large.

26. A nanotransport system as claimed in claim 25, **characterised in that** the system is larger than 15 nm.

27. A method of producing nanotransport systems with a dendritic architecture as claimed in one of claims 1 to 26, **characterised in that** the modular building up of the shells occurs from the exterior to the interior.

28. Use of the nanotransport system for transporting pharmaceutically active substances, particularly chemotherapeutic agents, cytostatic agents, antibodies, peptides and nucleic acids.

29. Use of the nanotransport system as claimed in one of claims 1 to 26 for producing a means for transporting chemical substances, particularly colouring agents or visual effect agents.

30. Use of the nanotransport system as claimed in one of claims 1 to 26 for producing a means for the targeted liberation of pharmaceutically active substances, particularly in cancer therapy.

31. Use of the nanotransport system as claimed in one of claims 1 to 26 for producing a means for gene therapy, diagnostics and analytics.

32. Use of the nanotransport system as claimed in one of claims 1 to 26 for producing a means for transporting siRNA or other nucleic acid derivatives.

33. Use of the nanotransport system as claimed in one of claims 1 to 26 for producing a means for transporting radioactively marked active substances or molecules.

34. Use of the nanotransport system as claimed in one claims 1 to 26 for producing a means for application in humans and animals, particularly for intravenous application.

35. Use of the nanotransport system in diagnostics outside the human or animal body.

## Revendications

1. Système de nanotransport à architecture dendritique (arborescente), le système étant composé d'un coeur dendritique et d'au moins deux coques, **caractérisé en ce que** le système est monomoléculaire, une coque intérieure étant reliée au coeur dendritique au moyen d'un linker 1 et une coque extérieure étant reliée à la coque intérieure au moyen d'un linker 2.

2. Système de nanotransport selon la revendication 1, **caractérisé en ce que** les coques présentent des polarités différentes.

3. Système de nanotransport selon l'une des revendications précédentes, **caractérisé en ce que** le système présente une coque polaire et une coque non polaire.

4. Système de nanotransport selon l'une des précédentes revendications 1 à 3, **caractérisé en ce que** le système présente une coque intérieure non polaire et une coque extérieure polaire.

5. Système de nanotransport selon l'une des précédentes revendications 1 à 3, **caractérisé en ce que** le système présente une coque intérieure polaire et une coque extérieure non polaire.

6. Système de nanotransport selon l'une des précédentes revendications 1 ou 2, **caractérisé en ce que** les coques intérieure et extérieure sont polaires.

7. Système de nanotransport selon l'une des précédentes revendications 1 ou 2, **caractérisé en ce que** les coques intérieure et extérieure sont non polaires.

8. Système de nanotransport selon l'une des revendications précédentes, **caractérisé en ce que** le système présente un coeur dendritique, celui-ci étant composé de polymères dendritiques.

9. Système de nanotransport selon l'une des revendications précédentes, **caractérisé en ce que** le coeur dendritique est constitué de polyglycérine, polyamide, polyamine, polyéther ou polyester.

10. Système de nanotransport selon l'une des revendications précédentes, **caractérisé en ce que** le coeur dendritique est polarisé en fonction de sa modification.

11. Système de nanotransport selon la revendication 1, **caractérisé en ce que** la coque intérieure est constituée de chaînes alkyl longues ou cycliques, de préférence avec une longueur de carbone de C₂ - C₄₀, de plus grande préférence C₁₂ - C₄₀, d'anneaux aromatiques ou de stéroïdes.

12. Système de nanotransport selon la revendication 1, **caractérisé en ce que** la coque extérieure est constituée de polyéthylène glycol de formule structurelle (-CH₂CH₂O-)ₙ avec n = 3 - 150, de polyglycérine linéaire ou ramifiée (C₃H₂OH)ₙ avec n = 2 - 150, de polyglycérine-dendrone [G1 - G6], de polyéther, de saccharides ou de polyester.

13. Système de nanotransport selon l'une des revendications précédentes, **caractérisé en ce que** la coque extérieure est biocompatible.

14. Système de nanotransport selon l'une des revendications précédentes, **caractérisé en ce que** le linker 1 est fissible par des enzymes, sensible aux acides, sensible à la lumière ou sensible à la chaleur.

15. Système de nanotransport selon l'une des revendications précédentes, **caractérisé en ce que** le linker 1 est constitué de groupes ester, amide, acétal, imine, disulfure ou hydrazone.

16. Système de nanotransport selon l'une des revendications précédentes, **caractérisé en ce que** le linker 2 est fissible par des enzymes, sensible aux acides, sensible à la lumière ou sensible à la chaleur.

17. Système de nanotransport selon l'une des revendications précédentes, **caractérisé en ce que** le linker 2 est constitué de groupes ester, amide, acétal, disulfure ou éther.

18. Système de nanotransport selon l'une des revendications précédentes, **caractérisé en ce que** la coque extérieure est terminée par des groupes fonctionnels.

19. Système de nanotransport selon la revendication 18, **caractérisé en ce que** les groupes fonctionnels terminaux sont de préférence des groupes hydroxy ou alcoxy, de plus grande préférence amines, amides ou thiols.

20. Système de nanotransport selon l'une des revendications précédentes, **caractérisé en ce que** les groupes fonctionnels terminaux présentent une fonction dirigeante.

21. Système de nanotransport selon la revendication 20, **caractérisé en ce que** les groupes terminaux dirigeants sont de préférence des peptides, des anticorps, des oligosaccharides, des acides nucléiques ou des nucléotides.

22. Système de nanotransport selon l'une des revendications précédentes, **caractérisé en ce que** le système reçoit des substances polaires et/ou non polaires.

23. Système de nanotransport selon l'une des revendications précédentes, **caractérisé en ce que** le système agrège.

24. Système de nanotransport selon l'une des revendications précédentes, **caractérisé en ce que** le système a une taille de 3 - 50 nm, de préférence 3 - 25 nm.

25. Système de nanotransport selon l'une des revendications précédentes, **caractérisé en ce que** le coeur dendritique a une taille de 3 - 15 nm.

26. Système de nanotransport selon la revendication 25, **caractérisé en ce que** le système est plus gros que 15 nm.

27. Procédé de fabrication de systèmes de nanotransport à architecture dendritique selon l'une des revendications 1 à 26, **caractérisé en ce que** la constitution modulaire des coques s'effectue de l'extérieur vers l'intérieur.

28. Utilisation du système de nanotransport pour le transport de principes pharmaceutiques actifs, en particulier de produits de chimiothérapie, de cytostatiques, d'anticorps, de peptides et d'acides nucléiques.

29. Utilisation du système de nanotransport selon l'une des revendications 1 à 26 pour fabriquer un moyen de transport de produits chimiques, en particulier de colorants ou de substances à effet.

30. Utilisation du système de nanotransport selon l'une des revendications 1 à 26 pour fabriquer un moyen de libération ciblée de principes pharmaceutiques actifs, en particulier dans le traitement du cancer.

31. Utilisation du système de nanotransport selon l'une des revendications 1 à 26 pour fabriquer un moyen pour la thérapie génique, le diagnostic et l'analyse.

32. Utilisation du système de nanotransport selon l'une des revendications 1 à 26 pour fabriquer un moyen de transport de siRNA ou d'autres dérivés d'acides nucléiques.

33. Utilisation du système de nanotransport selon l'une des revendications 1 à 26 pour fabriquer un moyen de transport de principes actifs ou molécules marqués radioactivement.

34. Utilisation du système de nanotransport selon l'une des revendications 1 à 26 pour fabriquer un moyen d'application chez l'homme et l'animal, en particulier pour une application intraveineuse.

35. Utilisation du système de nanotransport en diagnostic à l'extérieur du corps humain ou animal.
